# FASCICULE DE BREVET EUROPEEN

(11) **EP 1 298 135 B1**
(45) Date de publication et mention de la délivrance du brevet: **16.08.2006**
(21) Numéro de dépôt: 02292148.0
(22) Date de dépôt: 30.08.2002
(51) Int. Cl.: C07F 7/08, A61K 8/18

(54) **Composition tinctoriale contenant un composé para-aminophénol ou paraphènylène diamine substitué par un radical silanique**
Silylsubstituierte p-Aminophenol oder p-Phenylendiamine enthaltende Färbemittel
Dyeing composition containing silyl substituted p-aminophenol or p-phenylenediamine

(30) Priorité: 28.09.2001 FR 0112523
(43) Date de publication de la demande: 02.04.2003
(73) Titulaire: L'ORÉAL, 75008 Paris (FR)
(72) Inventeur: Richard, Hervé, 93420 Villepinte (FR)
(74) Mandataire: Fevrier, Murielle Françoise E.

(56) Documents cités:
- WO-A-01/66069
- DE-U- 20 111 038
- US-B1- 6 203 578

## Description

L'invention a pour objet une composition tinctoriale comprenant une base d'oxydation particulière du type paraphènylènediamine ou paraminophénol, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Le document WO 01/66 069 decrit une composition de teinture contenant des derivés para-phenylene diamine substitués sur un des atomes d'azote par des substituants autres que silanique.

Le but de la présente invention est de fournir de nouvelles compositions pour la teinture de fibres kératiniques par oxydation à partir de base d'oxydation particulièrement réactives et qui présentent des teintures puissantes, peu sélectives et particulièrement résistantes aux agents extérieurs tels que les shampoings et la lumière, et capables d'engendrer des colorations intenses dans des nuances variées.

Ce but est atteint avec la présente invention qui a pour objet une composition tinctoriale comprenant à titre de base d'oxydation au moins un composé répondant à la formule suivante (I) ainsi que les sels cosmétiquement acceptables correspondants : 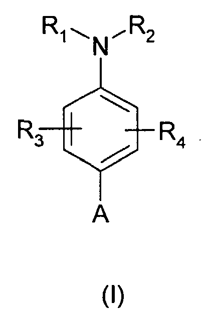 dans laquelle :
- A représente OH ou NH₂,
- R₁ et R₂, identiques ou différents, désignent l'hydrogène ; un radical alkyle en C₁-C₈, linéaires ou ramifiés ; un radical hydroxyalkyle en C₁-C₆ ou le radical -Y-B ; R₁ et R₂, ensemble forment avec l'atome d'azote auquel ils sont rattachés un hétérocycle à 4 à 7 chaînons, éventuellement substitué par un ou plusieurs radicaux alkyles en C₁-C₃, OH, NH₂ ou Z-B ; avec la condition que lorsque A représente OH, alors R₁ et R₂ désignent l'hydrogène,
- R₃ et R₄, identiques ou différents, désignent l'hydrogène, un radical alkyle en C₁-C₄, un radical alcoxy en C₁-C₄ ; un halogène ou le radical -Z-B,
- Y représente une chaîne hydrocarbonée en C₁-C₈ saturée ou insaturée, linéaire ou ramifiée pouvant être interrompue par un hétéroatome choisi parmi O, S ou NR, R étant un atome d'hydrogène ou un radical alkyle en C₁-C₄,
- Z représente une chaîne hydrocarbonée en C₁-C₈ saturée ou insaturée, linéaire ou ramifiée pouvant être interrompue par un hétéroatome choisi parmi O, S ou NR', R' étant un atome d'hydrogène ou un radical alkyle en C₁-C₄ ; un radical O-Y ou un radical NR"-Y dans laquelle R" représente un atome d'hydrogène ou un radical alkyle en C₁₋C₄,
- B est le groupe -SiR₅R₆R₇ dans lequel R₅, R₆ et R₇, identiques ou différent représentent un radical alkyle en C₁-C₄ ou un radical phényle,
à la condition qu'au moins un des groupes R₁, R₂, R₃ et R₄ représente -Y-B ou -Z-B.

Dans les définitions ci dessus, les radicaux ou groupes alkyle sont linéaires ou ramifiés et comprennent, sauf autre indication, de 1 à 8 atomes de carbone, de préférence 1 à 6 atomes de carbone, par exemple méthyle, éthyle, n-propyle, isopropyle, butyle, etc

Un radical alcoxy est un radical -O-Alkyl, le terme alkyle étant tel que défini précédemment.

Selon un mode de réalisation particulier, R₁ est un atome d'hydrogène et R₂, R₃ et R₄ sont tels que définis ci dessus, et de préférence R₃ et/ou R₄ représentent un atome d'hydrogène.

Selon un second mode de réalisation, R₁ et R₂ sont des atomes d'hydrogène et le ou les groupes -Z-B sont sur le cycle benzénique.

Selon un autre mode de réalisation, R₁ et R₂ forment avec l'atome d'azote auquel ils sont rattachés un hétérocycle à 5 ou 6 chaînons, l'hétérocycle étant substitué par un groupe -Z-B ou, et R₃ et R₄ étant tels que définis précédemment. Selon ce mode de réalisation particulier, R₃ et R₄ sont de préférence des atomes d'hydrogène.

A titre d'exemple, on peut citer

| | |
|---|---|
| 2-triméthylsilanylméthoxy-paraphénylènediamine | 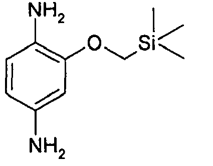 |
| N-triméthylsilanylméthyl-paraphénylènediamine : | 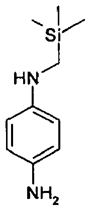 |
| 2-[(triméthylsilanyl)-éthyl]-paraphénylènediamine : | 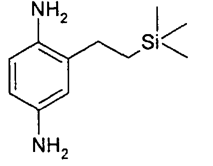 |
| N-triméthylsilanylpropyl-paraphénylènediamine : | 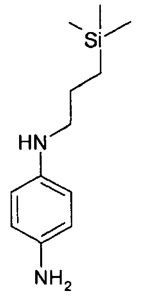 |
| 2-(triméthylsilanyl)-paraphénylènediamine: | 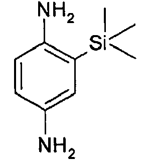 |
| 2,5-di-(triméthylsilanyl)-paraphénylènediamine : | 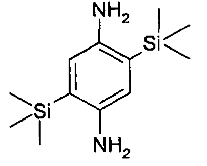 |
| 2-triméthylsilanyléthoxy-paraphénylènediamine : | 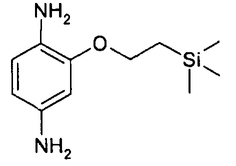 |
| 4-amino-3-(triméthylsilanylméthyl)-phénol : | 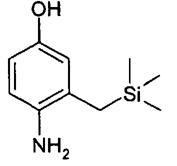 |
| 4-amino-3-(triméthylsilanyl)-phénol : | 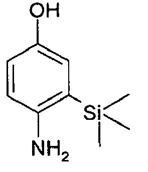 |

Selon la présente invention, le composé de formule (I) est généralement présent dans la composition tinctoriale de l'invention en quantité comprise entre 0,0005 et 12 % en poids environ du poids total de la composition tinctoriale, de préférence comprise entre 0,005 et 6 % en poids de ce poids.

Les composés de la présente invention peuvent être obtenus à partir des procédés de préparation généraux connus de l'homme du métier, par exemple par réduction des composés para-nitro-anilines silaniques ou para-nitrophénols silaniques correspondants.

Cette étape de réduction permet d'obtenir une amine aromatique primaire qui confère au composé synthétisé son caractère de de base d'oxydation. Cette étape est suivie ou non d'une salification.

Par commodité, cette étape est en général la dernière étape de la synthèse. Cependant, cette réduction peut intervenir plus tôt dans la suite des réactions conduisant à la préparation des composés de formule (I). Dans ce cas, selon des procédés bien connus, il faut alors protéger l'amine primaire créée, par exemple par une étape d'acétylation, de benzènesulfonation, etc..., et faire ensuite la ou les substitutions ou modifications désirées par exemple une alkylation, une réaction de Grignard, des réactions de substitutions nucléophiles avec le ou les dérivés silaniques, ou d'autres réactions bien connues de l'homme du métier, et terminer par la déprotection de la fonction amine, en général en milieu acide.

La composition tinctoriale de la présente invention est une composition cosmétique particulièrement utile pour la teinture des fibres kératiniques, en particuliers des fibres kératiniques humaines telles que les cheveux. Elle peut aussi être utile dans le domaine du maquillage.

En plus des composés précédemment décrits, la composition de la présente invention peut contenir une base d'oxydation additionnelle choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques ainsi que les sels cosmétiquement acceptables de ces composés.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, la 2 thiényl paraphénylène diamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut de plus contenir un ou plusieurs coupleurs additionnels qui sont conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques ainsi que les sels cosmétiquement acceptables de ces composés.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont présents en quantité de préférence comprise entre 0,001 et 10 % en poids environ du poids total de la composition tinctoriale et encore plus préférentiellement de 0,005 à 6 %, et la ou les bases d'oxydation additionnelles sont présentes en quantité de préférence comprise entre 0,001 à 10 % en poids environ du poids total de la composition tinctoriale, et encore plus préférentiellement de 0,005 à 6 %.

D'une manière générale, les sels cosmétiquement utilisables dans le cadre de la composition de l'invention pour les bases d'oxydation et les coupleurs présents sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les les sels d'addition avec une base telles que la soude, la potasse, l'ammoniaque, les amines ou les alcanolamines.

La composition conforme à l'invention peut en outre contenir un ou plusieurs colorants directs pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 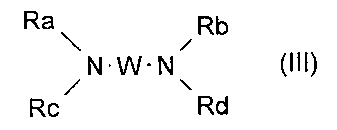 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition selon la présente invention telle que définie précédemment, la couleur étant révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale définie ci-dessus et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913au nom de la demanderesse.

Enfin l'invention a également pour objet le produit coloré résultant de l'oxydation de la composition selon la présente invention. Ces produits colorés peuvent notamment se présenter sous la forme de pigments et être utilisés à titre de colorant direct pour la teinture directe des cheveux ou bien encore être incorporés dans des produits cosmétiques tels que par exemple dans des produits de maquillage.

Les exemples qui suivent servent à illustrer l'invention sans toutefois présenter un caractère limitatif.

### EXEMPLES

### EXEMPLES DE SYNTHESES

### EXEMPLE 1 : Synthèse du dichlorhydrate de 2-triméthylsilanylméthoxy-para-phénylènediamine :

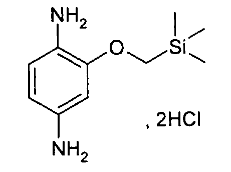

### a/ Première étape : synthèse du N-(4-nitro-2-triméthylsilanylméthoxy-phényl)-acétamide

On porte à 65°C sous agitation un mélange de 84,9 g (0,433 mole) de N-(2-hydroxy-4-nitro-phényl)-acétamide et de 144 g (0,44 mole) de carbonate de césium dans 350 ml de DMF anhydre, sous atmosphère d'azote. On ajoute à ce mélange hétérogène en 4 heures, sous agitation, goutte à goutte et à 65°C, 58,4 g (0,476 mole) de chlorométhyltriméthylsilyle. On refroidit et on verse le mélange réactionnel dans 700 ml d'eau glacée sous agitation. Le précipité formé est filtré, lavé à l'eau et séché. On obtient 119 g (Rendement de 97%) de N-(4-nitro-2-triméthylsilanylméthoxy-phényl)-acétamide sous forme d'une poudre brun clair.
Point de fusion : 128°C.
Ce produit est utilisé tel quel dans l'étape suivante.

### b/ Deuxième étape : synthèse du 4-nitro-2-triméthylsilanylméthoxy-phénylamine

On solubilise à chaud (65°C) sous agitation et sous atmosphère d'azote 104 g (0,368 mole) du produit précédent dans 600 ml de méthanol. On ajoute goutte à goutte au mélange réactionnel et en 2 heures 30 minutes une solution de méthylate de sodium dans le méthanol (3,2 g de méthylate de sodium à 30% sont dilué dans 80 ml de méthanol, soit 17 méq de méthylate de sodium). Le mélange est refroidi et est versé dans 2 litres d'eau sous agitation. Le solide jaune formé est filtré, lavé à l'eau et séché sous vide. On obtient 95,5 g (Rendement de 99%) de 4-nitro-2-triméthylsilanylméthoxyphénylamine sous forme de paillettes jaunes.
Point de fusion : 87°C.
Ce produit est utilisé tel quel dans l'étape suivante.

### c/ Troisième étape : synthèse du dichlorhydrate de 2-triméthylsilanylméthoxy-para-phénylènediamine

Dans un hydrogénateur de 500 ml, on introduit 35,7 g (0,138 mole) du dérivé précédent, 3,5g de palladium sur charbon à 5% mouillé avec 50% d'eau et 350 ml d'éthanol absolu. On ferme l'hydrogénateur et sous une pression d'hydrogène (5 bars), on maintient le mélange à 40°C pendant 1 heure. Après dégazage à l'azote, on verse le mélange réactionnel dans 200 ml d'éthanol contenant 26 ml d'acide chlorhydrique concentré (soit 280 méq). Le solvant est évaporé à sec et séché sous vide. On obtient ainsi 38,8 g (Rendement de 99%) de produit sous forme d'une poudre blanc cassé.

| Analyse élémentaire pour C₁₀H₁₆ N₂O Si 2HCl | | | | | |
|---|---|---|---|---|---|
| calculé : | C 42,40 ; | H 7,12 ; | N 9,89 ; | Si 9,91 ; | Cl 25,03 |
| trouvé : | C 42,52 ; | H 7,07 ; | N 9,71 ; | Si 9,50 ; | Cl 25,25 |

### EXEMPLE 2 : Synthèse du dichlorhydrate de N-triméthylsilanylméthyl-para-phénylènediamine :

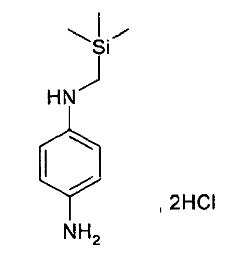

### a/ Première étape : Synthèse du N-(4-nitro-phényl)-N-triméthylsilanylméthyl-amine

On ajoute goutte à goutte en 15 minutes à la température ambiante 5 g (0,048 mole) d'aminométhyltriméthylsilane à un mélange de 6,83 g (0,048 mole) de 1-fluoro-4-nitrobenzène et de 6,68 ml (0,048 mole) de triéthylamine dissout dans 25 ml de dichlorométhane. On laisse sous agitation pendant 6 heures. On ajoute 50 ml d'eau et sépare les 2 phases. On extrait la phase aqueuse par du dichlorométhane. Les phases organiques sont regroupées, lavées à l'eau, séchées sur sulfate de sodium, filtrées puis concentrées. L'huile orangée obtenue est purifiée sur silice (éluant : Heptane/AcOEt 95/5) pour donner des fractions propres qui cristallisent de N-(4-nitrophényl)-N-triméthylsilanylméthyl-amine : poids : 4,15 g (Rendement de 38%) : Poudre jaune.
Point de fusion : 120°C.
Spectre de masse : M⁺+H = 225.

### a/ Deuxième étape : Synthèse du dichlorhydrate de N-triméthylsilanylméthyl-para-phénylènediamine

Dans un réacteur sous bullage d'argon, on introduit 2,29 g (35 mmole) de zinc en poudre, 117 mg (2,18 mmol) de chlorure d'ammonium, 7 ml d'éthanol à 96° et 1 ml d'eau distillée. On chauffe ce mélange au reflux de l'éthanol. On ajoute portion par portion en 30 minutes 1 g (4,45 mmole) du produit de l'étape précédente. On maintient sous bon reflux pendant 30 minutes. On filtre le mélange réactionnel à chaud et on ajoute rapidement 0,3 ml d'acide chlorhydrique concentré au filtrat. Ce dernier de couleur violette puis jaune pâle est évaporé à sec, trituré dans du cyclohexane et séché sous vide en présence de pastilles de potasse à 25°C. On obtient ainsi 0,87 g (Rendement de 58%) de produit sous forme d'une poudre jaune pâle.

| Analyse élémentaire pour C₁₀ H₁₈ N₂ Si .2HCl | | | | | |
|---|---|---|---|---|---|
| calculé : | C 44,94 ; | H 7,54 ; | N 10,48 ; | Si 10,51 ; | Cl 26,53 |
| trouvé : | C 44,42 ; | H 7,27 ; | N 10,58 ; | Si 10,70 ; | Cl 26,07 |

### EXEMPLE 3 : Synthèse du dichlorhydrate de 2-[(triméthylsilanyl)-éthyl]-para-phénylènediamine :

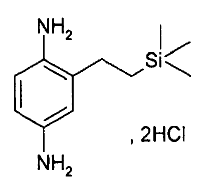

### a/ Première étape : Synthèse de 2-iodo-4-nitro-aniline

On dissout à chaud (vers 80°C) 42,7 g (0,309 mole) de 4-nitroaniline dans 150 ml d'acide acétique. On y ajoute en 30 minutes, goutte à goutte, une solution de 50 g (0,309 mole) d'iodine monochloride dans 100 ml d'acide acétique. On laisse sous agitation à 100°C pendant 2 heures. Par CCM, il reste une trace de produit de départ. On ajoute donc 1 g d'iodine monochloride et laisse à 100°C sous agitation pendant 1 heure. Le mélange réactionnel est filtré à chaud. Le solide est lavé à l'éther puis séché. On obtient ainsi 74 g d'une poudre gris vert de 2-iodo-4-nitro-aniline (Rendement de 91%). Ce produit est utilisé tel quel dans l'étape suivante.

### b/ Deuxième étape : Synthèse du 4-nitro-2-[(triméthylsilanyl)-éthynyl]-phénylamine

Dans un réacteur sous bullage d'argon on introduit 19,8 g (0,075 mole) du dérivé précédent, 1,6 g (2,25 mmole) de Bis(triphénylphosphine)-palladium(II)-chloride, 0,86 g (4,5 mmole) d' iodure cuivreux dans 3001 de triéthylamine. On refroidit vers 3°C et on introduit par portions en 20 minutes sous atmosphère d'argon 13 ml (0.09 mole) de (triméthylsilyl)éthyne. On laisse à cette température sous agitation pendant 1 heure. Le solvant est évaporé sous vide. Le résidu est solubilisé à chaud dans l'éthanol et traité au charbon actif. On filtre sur Célite et on évapore le solvant. Le résidu est repris dans le diéthyl éther et le résidu éliminé par filtration. La phase organique est lavée avec une solution aqueuse de chlorure de sodium. Elle est séchée et le solvant est évaporé sous vide. Le solide obtenu est recristallisé dans l'heptane pour donner 8,1 g (Rendement 47%) de 4-nitro-2-[(triméthylsilanyl)-éthynyl]-phénylamine sous forme de cristaux jaunes. Ce produit est utilisé tel quel dans l'étape suivante.

### c/ Troisième étape : Synthèse du dichlorhydrate de 2-[(triméthylsilanyl)-éthyl]-para-phénylènediamine

Dans un hydrogénateur de 250 ml, on introduit 6,75 g (0,029 mole) du dérivé précédent, 0,68 g de palladium sur charbon à 5% mouillé avec 50% d'eau et 120 ml d'éthanol absolu. On ferme l'hydrogénateur et sous une pression d'hydrogène (5 bars), on maintient le mélange à 60°C pendant 4 heures. Après dégazage à l'azote, on verse le mélange réactionnel dans 75 ml d'éthanol contenant 6 ml d'acide chlorhydrique concentré (soit 66 méq). Le solvant est évaporé à sec et séché sous vide. On obtient ainsi 6,75 g (Rendement de 84%) du produit de l'exemple 1 sous forme d'une poudre blanc cassé.
Point de fusion : 140°C (début décomposition)-160°C.

| Analyse élémentaire pour C₁₁H₂₀N₂ Si .2HCl | | | | | |
|---|---|---|---|---|---|
| calculé : | C 46,97 ; | H 7,88 ; | N 9,96 ; | Si 9,98 ; | Cl 25,21 |
| trouvé : | C 46,43 ; | H 7,40 ; | N 10,43 ; | Si 9,45 ; | Cl 25,80 |

### EXEMPLE 4 : Synthèse du dichlorhydrate de N-triméthylsilanylpropyl-para-phénylènediamine :

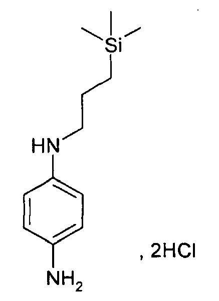

### a/ Première étape : Synthèse du chlorure de l'acide 3-triméthylsilyl propionique

On introduit goutte à goutte à température ambiante, 13,5g (0,11 mole) de chlorure de thionyle à une solution de 10 g (0,068 mole) d'acide 3-triméthylsilyl propionique dans 50 ml de dichloro-1,2 éthane. Le mélange est chauffé à 40°C pendant 2 heures et l'excès de chlorure de thionyle est distillé sous vide. On obtient une solution de chlorure de l'acide 3-triméthylsilyl propionique dans le dichloro-1,2 éthane utilisée telle quelle dans l'étape suivante.

### b/ Deuxième étape : Synthèse du N-(4-nitro-phényl)-3-triméthylsilanyl-propionamide

On dissout 9,3 g (0,067 mole) de 4-nitroaniline et 7,1 g (0,07 mole) de triéthylamine dans 50 ml de dichloro-1,2 éthane. On y ajoute en 10 minutes, goutte à goutte la solution obtenue précédemment. On laisse sous agitation à 50°C pendant 2 heures. Le mélange réactionnel est versé dans l'eau et la phase organique est séparée, lavée à l'eau acidulée, puis avec une solution bicarbonatée, enfin à l'eau. Après séparation de la phase organique, séchage sur sulfate de sodium et évaporation du solvant, on obtient une huile brune. Elle est traitée au charbon actif au reflux de l'éthanol pour la décolorer. Le nouveau brut obtenu est purifié sur silice (éluant : AcOEt/Heptane 1 :2) pour obtenir 12,5 g d'une poudre grise de N-(4-nitro-phényl)-3-triméthylsilanylpropionamide (Rendement de 70%). Ce produit est utilisé tel quel dans l'étape suivante.

### c/ Troisième étape : Synthèse du (4-nitro-phényl)-(3-triméthylsilanyl-propyl)-amine

Sous bullage d'argon 9,5g (0,036 mole) du produit précédent sont dissout dans 300ml de THF anhydre. Le milieu est refroidit à 0°C et 2,1g d'hydrure de lithium et d'aluminium sont ajoutés par portions. Le mélange est laissé 1 heure à 0°C puis 2 heures au reflux. Après refroidissement, le mélange réactionnel est hydrolysé avec 25ml d'un mélange THF/eau puis de l'eau à chaud. Après filtration, le filtrat est évaporé. L'huile noire obtenue est purifié par chromatographie préparative sur silice (éluant : AcOEt/Heptane 1 : 4). On récupère 2,1g (Rendement de 23%) de fractions pures de (4-nitro-phényl)-(3-triméthylsilanyl-propyl)-amine sous forme d'une huile orangée.

### d/ Quatrième étape : Synthèse du dichlorhydrate de N-triméthylsilanylpropyl-para-phénylènediamine_

Dans un hydrogénateur de 250 ml, on introduit 1,8 g (0,00713 mole) du dérivé précédent, 0,2 g de palladium sur charbon à 5% mouillé avec 50% d'eau et 120 ml d'éthanol absolu. On ferme l'hydrogénateur et sous une pression d'hydrogène (4 bars), on maintient le mélange à 40°C pendant 4 heures. Après dégazage à l'azote, on verse le mélange réactionnel dans 80 ml d'éthanol contenant 1,5 ml d'acide chlorhydrique concentré (soit 23 méq). Le solvant est évaporé à sec et séché sous vide. Le solide obtenu est lavé à l'acétate d'éthyle puis séché. On obtient ainsi 1,1 g (Rendement de 52%) du produit de l'exemple 4 sous forme d'une poudre beige pâle.

| Analyse élémentaire pour C12 H22 N2 Si .2HCl | | | | | |
|---|---|---|---|---|---|
| calculé : | C 48,8 ; | H8,19 ; | N9,49 ; | Si9,51 ; | Cl24,01 |
| trouvé : | C 48,96 ; | H 8,00 ; | N 9,44 ; | Si 9,12 ; | Cl 23,56 |

### EXEMPLE 5 : Synthèse du chlorhydrate de 4-amino-3-(triméthylsilanylméthyl)-phénol :

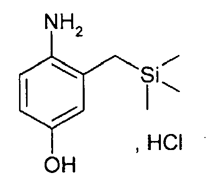

### a/ Première étape : Synthèse du 4-nitro-triméthylsilyloxy-benzène

Sous barbotage d'argon, 15g (0,138 mole) de 4-nitrophénol et 7,5g (0,074 mole) de triéthylamine sont dissout dans 30 ml de THF. On y introduit goutte à goutte à température ambiante, 15g (0,138 mole) de chlorure de triméthylsilyle. Ce mélange est porté au reflux pendant 2 heures 30 minutes. Le mélange réactionnel est refroidi. Le chlorhydrate de triéthylamine est séparé par filtration. Le solvant est éliminé par distillation sous pression atmosphérique. Après distillation sous pression réduite (0,3 mmHg) de l'huile obtenue, on récupère 12,1g (Rendement de 85%) des fractions distillants à 96-110°C. Ce produit est utilisé tel quel dans l'étape suivante.

### b/ Deuxième étape : Synthèse du 4-nitro-3-triméthylsilanylméthyl-phénol

Le magnésien du chlorométhyltriméthylsilane (réactif de Peterson) est d'abord préparé de la manière suivante : Sous gaz inerte argon, 2,7g de tournures de magnésium (0,111 mole) sont recouvertes avec un minimum de THF. Quelques cristaux d'iode sont introduits ainsi qu'un vingtième de la quantité de chlorométhyltriméthylsilane (quantité totale : 13,3g, 0,108 mole). Quelques gouttes de 1,2-dibromoéthane sont introduites et de nouveau un vingtième de la quantité de chlorométhyltriméthylsilane est introduite. Après chauffage, la réaction démarre. Le bain chauffant est baissé et le reste de chlorométhyltriméthylsilane dissout dans 60 ml de THF est introduit goutte à goutte dans le mélange réactionnel maintenu au reflux. On laisse ce mélange au reflux pendant 2 heures après la fin de l'introduction, puis le refroidi.
Dans un autre ballon, 10,1g (0,048 mole) du produit de l'étape 1 sont dissout dans 150ml de THF. Ce mélange sous atmosphère d'argon est refroidit à -78°C à l'aide d'un bain de carboglace. On y introduit en 1 heure à l'aide d'une canule entre les 2 ballons le magnésien précédent en poussant à l'argon. La nitrone formée est ensuite réaromatisée à l'aide de l'introduction goutte à goutte de 19g (0,86 mole) de 2,3-dichloro 5,6-dicyano quinone (DDQ) dissout dans 150ml de THF en 20 minutes. La température du mélange réactionnel est ensuite progressivement ramenée à température ambiante. Ce mélange est versé dans 380ml d'acide acétique à 5% puis est extrait au dichlorométhane. La phase organique est lavée avec une solution de bicarbonate de sodium puis à l'eau. Après séchage avec du sulfate de sodium et filtration, le solvant est évaporé sous vide. Le brut obtenu est extrait à chaud avec de l'heptane pour donner une huile marron après évaporation du solvant. Le nouveau brut obtenu est purifié par chromatographie préparative sur silice (éluant : AcOEt/Heptane 1 :3). On récupère 6,1g (Rendement de 57%) de fractions pures de 4-nitro-3-triméthylsilanylméthyl-phénol.

### c/ Troisième étape : Synthèse du chlorhydrate de 4-amino-3-(triméthylsilanylméthyl)-phénol :

Dans un hydrogénateur de 250 ml, on introduit 5,8 g (0,026 mole) du dérivé précédent, 0,6 g de palladium sur charbon à 5% mouillé avec 50% d'eau et 120 ml d'éthanol absolu. On ferme l'hydrogénateur et sous une pression d'hydrogène (6 bars), on maintient le mélange à 60°C pendant 4 heures. Après dégazage à l'azote, on verse le mélange réactionnel dans 80 ml d'éthanol contenant 3 ml d'acide chlorhydrique concentré (soit 36 méq). Le solvant est évaporé à sec et séché sous vide. Le solide obtenu est lavé à l'acétate d'éthyle puis séché. On obtient ainsi 2,75 g (Rendement de 46%) du produit de l'exemple 5 sous forme d'une poudre beige pâle.

| Analyse élémentaire pour C10 H17 N O Si .HCl | | | | | |
|---|---|---|---|---|---|
| calculé : | C 51,82 ; | H7,83 ; | N6,04 ; | Si12,12 ; | Cl15,29 |
| trouvé : | C 51,85 ; | H 7,77 ; | N 6,16 ; | Si 11,87 ; | Cl 15,64 |

### EXEMPLES DE TEINTURE

On a préparé les compositions tinctoriales suivantes (teneurs en mole):

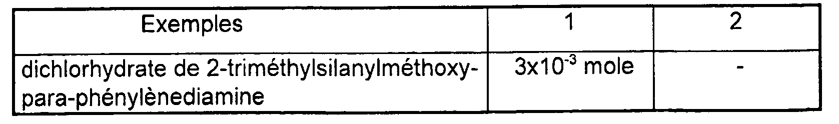

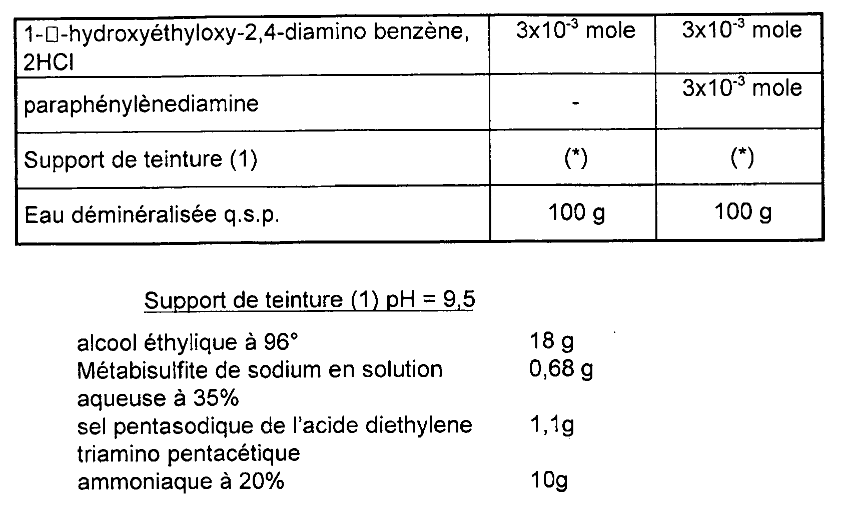

Au moment de l'emploi, chaque composition est mélangée avec un poids égal d'eau oxygénée à 20 volumes (6% en poids).

Chaque mélange obtenu est appliqué sur des mèches de cheveux gris à 90 % de blancs, permanentés (BP) ou naturel (BN). Après 30 min de pose, les mèches sont rinçées, lavées avec un shampooing standard, rinçées à nouveau puis séchées.

Chaque mèche est évaluée avant et après la teinture dans le système L*a*b*, au moyen d'un spectrophotomètre CM 2002 MINOLTA ®, (Illuminant D65).

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

Les résultats de teinture suivants ont été obtenus.

| | Cheveux naturels | | | Cheveux permanentés | | |
|---|---|---|---|---|---|---|
| | L^{*} | a^{*} | b^{*} | L^{*} | a^{*} | b^{*} |
| Exemple 1 | 22,3 | 0,08 | -5,4 | 19,7 | 0,8 | -2,3 |

Dans une deuxième série d'essais, on a mesuré la ténacité aux shampoings de la teinture obtenue à partir de l'exemple 1 (invention) et à partir de l'exemple 2 (témoin) sur cheveux permanentés.

Pour chaque mèche, on mesure les valeurs L*a*b* avant l'épreuve aux shampoings et après 8 shampooings.

Les résultats sont reportés dans le tableau suivant

| | Avant shampoing | | | Après 8 shampoings | | |
|---|---|---|---|---|---|---|
| | L^{*} | a^{*} | b^{*} | L^{*} | a^{*} | b^{*} |
| Exemple 1 | 18,6 | 0,2 | -1,3 | 19,3 | -0,1 | -1,1 |
| Exemple 2 | 18,7 | 0,6 | -2,2 | 27,1 | 2,6 | 1,3 |

## Revendications

1. Composition tinctoriale comprenant à titre de base d'oxydation au moins un composé répondant à la formule suivante (I) ainsi que les sels cosmétiquement acceptables correspondants : 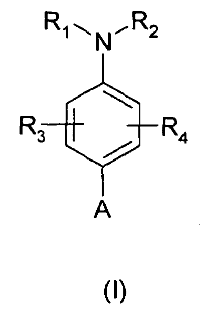 dans laquelle :
- A représente OH ou NH₂,
- R₁ et R₂, identiques ou différents, désignent l'hydrogène ; un radical alkyle en C₁-C₈, linéaires ou ramifiés ; un radical hydroxyalkyle en C₁-C₆ ou le radical -Y-B ; R₁ et R₂, ensemble forment avec l'atome d'azote auquel ils sont rattachés un hétérocycle à 4 à 7 chaînons, éventuellement substitué par un ou plusieurs radicaux alkyles en C₁-C₃, OH, NH₂ ou Z-B ; avec la condition que lorsque A représente OH, alors R₁ et R₂ désignent l'hydrogène,
- R₃ et R₄, identiques ou différents, désignent l'hydrogène, un radical alkyle en C₁-C₄, un radical alcoxy en C₁-C₄ ; un halogène ou le radical -Z-B,
- Y représente une chaîne hydrocarbonée en C₁-C₈ saturée ou insaturée, linéaire ou ramifiée pouvant être interrompue par un hétéroatome choisi parmi O, S ou NR, R étant un atome d'hydrogène ou un radical alkyle en C₁-C₄,
- Z représente une chaîne hydrocarbonée en C₁-C₈ saturée ou insaturée, linéaire ou ramifiée pouvant être interrompue par un hétéroatome choisi parmi O, S ou NR', R' étant un atome d'hydrogène ou un radical alkyle en C₁-C₄ ; un radical O-Y ou un radical NR"-Y dans laquelle R" représente un atome d'hydrogène ou un radical alkyle en C₁₋C₄,
- B est le groupe -SiR₅R₆R₇ dans lequel R₅, R₆ et R₇, identiques ou différent représentent un radical alkyle en C₁-C₄ ou un radical phényle, à la condition qu'au moins un des groupes R₁, R₂, R₃ et R₄ représente -Y-B ou -Z-B.

2. Composition selon la revendication 1 dans laquelle le composé de formule (I) est tel que R₁ est un atome d'hydrogène.

3. Composition selon la revendication 1 ou 2 dans laquelle le composé de formule (I) est tel que R₃ et/ou R₄ représentent un atome d'hydrogène.

4. Composition selon la revendication 1 dans laquelle le composé de formule (I) est tel que R₁ et R₂ représentent un atome d'hydrogène.

5. Composition selon la revendication 1 dans laquelle le composé de formule (I) est tel que R₁ et R₂ forment avec l'atome d'azote auquel ils sont rattachés un hétérocycle à 5 ou 6 chaînons, l'hétérocycle étant substitué par un groupe -Z-B.

6. Composition selon la revendication 5 dans laquelle le composé de formule (I) est tel que R₃ et R₄ sont des atomes d'hydrogène.

7. Composition selon l'une quelconque des revendications 1 à 6 dans laquelle le composé de formule (I) est choisi parmi les composés suivants ou les sels d'addition avec un acide de ces composés :
- la 2-triméthylsilanylméthoxy-para-phénylènediamine
- la N-triméthylsilanylméthyl-para-phénylènediamine
- la 2-[(triméthylsilanyl)-éthyl]-para-phénylènediamine
- la N-triméthylsilanylpropyl-para-phénylènediamine
- la 2-(triméthylsilanyl)-para-phénylènediamine
- la 2,5-di-(triméthylsilanyl)-para-phénylènediamine
- la 2-triméthylsilanyléthoxy-para-phénylènediamine
- le 4-amino-3-(triméthylsilanylméthyl)-phénol
- le 4-amino-3-(triméthylsilanyl)-phénol

8. Composition selon l'une quelconque des revendications précédentes dans laquelle la quantité de composé de formule (I) est comprise entre comprise entre 0,0005 et 12 % en poids environ du poids total de la composition tinctoriale.

9. Composition selon l'une quelconque des revendications 1 à 8 comprenant de plus une base d'oxydation choisie parmi les paraphénylènediamines, les bis-phénylalkylènediamines, les para-aminophénols, les ortho-aminophénols, les bases hétérocycliques, et leurs sels d'addition avec un acide ou une base.

10. Composition selon l'une quelconque des revendications 1 à 9 comprenant de plus un colorant direct.

11. Composition selon l'une quelconque des revendications 1 à 10 comprenant un agent oxydant.

12. Procédé de teinture d'oxydation des fibres kératiniques et en particulier des fibres kératiniques humaines telles que les cheveux, **caractérisé en ce qu'**on applique sur les fibres au moins une composition telle que définie à l'une quelconque des revendications 1 à 10, et qu'on révèle la couleur à l'aide d'un agent oxydant.

13. Procédé selon la revendication 12 dans lequel l'agent oxydant est choisi parmi le peroxyde d'hydrogène, le peroxyde d'urée, les bromates de métaux alcalins, les persels, les peracides et les enzymes oxydases.

14. Procédé selon l'une des revendications 12 ou 13 dans lequel l'agent oxydant est mélangé au moment de l'emploi à la composition telle que définie selon l'une quelconque des revendications 1 à 10.

15. Procédé selon l'une quelconque des revendications 12 ou 13 dans lequel l'agent oxydant est appliqué sous forme de composition oxydante simultanément ou séquentiellement à la composition telle que définie selon l'une quelconque des revendications 1 à 10 sur les fibres.

16. Dispositif à plusieurs compartiments ou "kit" de teinture à plusieurs compartiments, dans lequel un premier compartiment contient une composition telle que définie à l'une quelconque des revendications 1 à 10 et un deuxième compartiment contient une composition oxydante.

17. Composé de formule (I) tel que défini selon l'une quelconque des revendications 1 à 7.

18. Utilisation de la composition définie aux revendications 1 à 11 pour la tenture de fibres kératiniques.

## Claims

1. Dye composition comprising, as oxidation base, at least one compound corresponding to formula (I) below, and also the corresponding cosmetically acceptable salts: 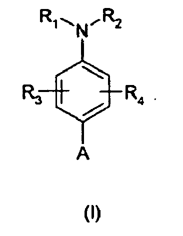 in which:
- A represents OH or NH₂,
- R₁ and R₂, which may be identical or different, denote hydrogen; a linear or branched C₁-C₈ alkyl radical; a C₁-C₆ hydroxyalkyl radical or the radical -Y-B; R₁ and R₂ form, together with the nitrogen atom to which they are attached, a 4- to 7-membered heterocycle, optionally substituted with one or more C₁-C₃ alkyl, OH, NH₂ or Z-B radicals; with the condition that when A represents OH, then R₁ and R₂ denote hydrogen,
- R₃ and R₄, which may be identical or different, denote hydrogen, a C₁-C₄ alkyl radical; a C₁-C₄ alkoxy radical; a halogen or the radical -Z-B,
- Y represents a linear or branched, saturated or unsaturated C₁-C₈ hydrocarbon-based chain that may be interrupted with a hetero atom chosen from O, S and NR, R being a hydrogen atom or a C₁-C₄ alkyl radical,
- Z represents a linear or branched, saturated or unsaturated C₁-C₈ hydrocarbon-based chain that may be interrupted with a hetero atom chosen from O, S and NR', R' being a hydrogen atom or a C₁-C₄ alkyl radical; a radical O-Y or a radical NR"-Y in which R" represents a hydrogen atom or a C₁-C₄ alkyl radical,
- B is the group -SiR₅R₆R₇ in which R₅, R₆ and R₇, which may be identical or different, represent a C₁-C₄ alkyl radical or a phenyl radical,
on condition that at least one of the groups R₁, R₂, R₃ and R₄ represents -Y-B or -Z-B.

2. Composition according to Claim 1, in which the compound of formula (I) is such that R₁ is a hydrogen atom.

3. Composition according to Claim 1 or 2, in which the compound of formula (I) is such that R₃ and/or R₄ represent a hydrogen atom.

4. Composition according to Claim 1, in which the compound of formula (I) is such that R₁ and R₂ represent a hydrogen atom.

5. Composition according to Claim 1, in which the compound of formula (I) is such that R₁ and R₂ form, with the nitrogen atom to which they are attached, a 5- or 6-membered heterocycle, the heterocycle being substituted with a group -Z-B.

6. Composition according to Claim 5, in which the compound of formula (1) is such that R₃ and R₄ are hydrogen atoms.

7. Composition according to any one of Claims 1 to 6, in which the compound of formula (I) is chosen from the following compounds, or the acid-addition salts of these compounds:
- 2-trimethylsilanylmethoxy-para-phenylenediamine
- N-trimethylsilanylmethyl-para-phenylenediamine
- 2-[(trimethylsilanyl)ethyl]-para-phenylenediamine
- N-trimethylsilanylpropyl-para-phenylenediamine
- 2-(trimethylsilanyl)-para-phenylenediamine
- 2,5-bis(trimethylsilanyl)-para-phenylenediamine
- 2-trimethylsilanylethoxy-para-phenylenediamine
- 4-amino-3-(trimethylsilanylmethyl)phenol
- 4-amino-3-(trimethylsilanyl)phenol.

8. Composition according to any one of the preceding claims, in which the amount of compound of formula (I) is between 0.0005% and 12% by weight approximately relative to the total weight of the dye composition.

9. Composition according to any one of Claims 1 to 8, also comprising an oxidation base chosen from para-phenylenediamines, bis(phenyl)alkylenediamines, para-aminophenols, ortho-aminophenols and heterocyclic bases, and the addition salts thereof with an acid or a base.

10. Composition according to any one of Claims 1 to 9, also comprising a direct dye.

11. Composition according to any one of Claims 1 to 10, comprising an oxidizing agent.

12. Process for the oxidation dyeing of keratin fibres and in particular of human keratin fibres such as the hair, **characterized in that** at least one composition as defined in any one of Claims 1 to 10 is applied to the fibres, and the colour is revealed using an oxidizing agent.

13. Process according to Claim 12, in which the oxidizing agent is chosen from hydrogen peroxide, urea peroxide, alkali metal bromates, persalts, peracids and oxidase enzymes.

14. Process according to either of Claims 12 and 13, in which the oxidizing agent is mixed at the time of use with the composition as defined according to any one of Claims 1 to 10.

15. Process according to either of Claims 12 and 13, in which the oxidizing agent is applied to the fibres in the form of an oxidizing composition simultaneously with or sequentially to the composition as defined according to any one of Claims 1 to 10.

16. Multi-compartment device or multi-compartment dyeing "kit", in which a first compartment contains a composition as defined in any one of Claims 1 to 10 and a second compartment contains an oxidizing composition.

17. Compound of formula (I) as defined according to any one of Claims 1 to 7.

18. Use of the composition defined in Claims 1 to 11, for dyeing keratin fibres.

## Patentansprüche

1. Färbezusammensetzung, die als Oxidationsbase mindestens eine Verbindung der nachstehenden Formel (I) sowie die entsprechenden kosmetisch akzeptablen Salze enthält: 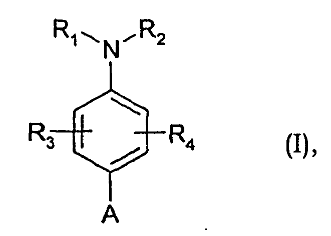 worin bedeuten:
- A OH oder NH₂,
- R₁ und R₂, die gleich oder verschieden sind, Wasserstoff; geradkettiges oder verzweigtes C₁-C₈-Alkyl; C₁-C₆-Hydroxyalkyl oder die Gruppe -Y-B; oder R₁ und R₂ bilden zusammen mit dem Stickstoffatom, an das sie gebunden sind, einen 4- bis 7-gliedrigen Heterocyclus, der gegebenenfalls mit einer oder mehreren C₁-C₃-Alkylgruppen, OH, NH₂ oder Z-B substituiert ist; mit der Maßgabe, dass, wenn A OH bedeutet, R₁ und R₂ Wasserstoff darstellen,
- R₃ und R₄, die gleich oder verschieden sind, Wasserstoff, C₁-C₄-Alkyl, C₁-C₄-Alkoxy; ein Halogen oder die Gruppe -Z-B,
- Y eine gesättigte oder ungesättigte, geradkettige oder verzweigte C₁-C₈-Kohlenwasserstoffkette, die mit einem Heteroatom unterbrochen sein kann, das unter O, S oder NR ausgewählt ist, wobei R ein Wasserstoffatom oder eine C₁-C₄₋Alkylgruppe bedeutet,
- Z eine gesättigte oder ungesättigte, geradkettige oder verzweigte C₁-C₈-Kohlenwasserstoffkette, die durch ein Heteroatom unterbrochen sein kann, das unter O, S oder NR' ausgewählt ist, wobei R' ein Wasserstoffatom oder eine C₁-C₄₋Alkylgruppe bedeutet; eine Gruppe O-Y oder eine Gruppe NR"-Y, worin R" ein Wasserstoffatom oder eine C₁-C₄-Alkylgruppe bedeutet,
- B die Gruppe -SiR₅R₆R₇, in der R₅, R₆ und R₇, die gleich oder verschieden sind, eine C₁-C₄-Alkylgruppe oder eine Phenylgruppe darstellen,
mit der Maßgabe, dass mindestens eine der Gruppen R₁, R₂, R₃ und R₄ -Y-B oder -Z-B darstellt.

2. Zusammensetzung nach Anspruch 1, bei der die Verbindung der Formel (I) eine Verbindung ist, bei der R₁ ein Wasserstoffatom ist.

3. Zusammensetzung nach Anspruch 1 oder 2, bei der die Verbindung der Formel (I) eine Verbindung ist, bei der R₃ und/oder R₄ ein Wasserstoffatom darstellen.

4. Zusammensetzung nach Anspruch 1, bei der die Verbindung der Formel (I) eine Verbindung ist, bei der R₁ und R₂ ein Wasserstoffatom darstellen.

5. Zusammensetzung nach Anspruch 1, bei der die Verbindung der Formel (I) eine Verbindung ist, bei der R₁ und R₂ zusammen mit dem Stickstoffatom, an das sie gebunden sind, einen 5- oder 6-gliedrigen Heterocyclus bilden, der mit einer Gruppe -Z-B substituiert ist.

6. Zusammensetzung nach Anspruch 5, bei der die Verbindung der Formel (I) eine Verbindung ist, bei der R₃ und R₄ Wasserstoffatome sind.

7. Zusammensetzung nach einem der Ansprüche 1 bis 6, bei der die Verbindung der Formel (I) unter den folgenden Verbindungen oder den Additionssalzen dieser Verbindungen mit einer Säure ausgewählt ist:
- 2-Trimethylsilanylmethoxy-p-phenylendiamin,
- N-Trimethylsilanylmethyl-p-phenylendiamin,
- 2-[(Trimethylsilanyl)ethyl]-p-phenylendiamin,
- N-Trimethylsilanylpropyl-p-phenylendiamin,
- 2-(Trimethylsilanyl)-p-phenylendiamin,
- 2,5-Di-(trimethylsilanyl)-p-phenylendiamin,
- 2-Trimethylsilanylethoxy-p-phenylendiamin,
- 4-Amino-3-(trimethylsilanylmethyl)-phenol,
- 4-Amino-3-(trimethylsilanyl)-phenol.

8. Zusammensetzung nach einem der vorhergehenden Ansprüche, bei der die Menge der Verbindung der Formel (I) im Bereich von etwa 0,0005 bis 10 Gew.-% liegt, bezogen auf das Gesamtgewicht der Färbezusammensetzung.

9. Zusammensetzung nach einem der Ansprüche 1 bis 8, die ferner eine Oxidationsbase enthält, die ausgewählt ist unter p-Phenylendiaminen, Bis-phenylalkylendiaminen, p-Aminophenolen, o-Aminophenolen und heterocyclischen Basen sowie den Additionssalzen dieser Verbindungen mit einer Säure oder einer Base.

10. Zusammensetzung nach einem der Ansprüche 1 bis 9, die ferner einen Direktfarbstoff enthält.

11. Zusammensetzung nach einem der Ansprüche 1 bis 10, die ein Oxidationsmittel enthält.

12. Verfahren zur Oxidationsfärbung von Keratinfasern und insbesondere menschlichen Keratinfasern wie der Haare, **gekennzeichnet durch** Aufbringen mindestens einer Zusammensetzung, wie sie in einem der Ansprüche 1 bis 10 definiert ist, auf die Fasern und Entwickeln der Farbe mit einem Oxidationsmittel.

13. Verfahren nach Anspruch 12, bei dem das Oxidationsmittel unter Wasserstoffperoxid, Harnstoffperoxid, Alkalimetallbromaten, Persalzen, Persäuren und Oxidase-Enzymen ausgewählt ist.

14. Verfahren nach Anspruch 12 oder 13, bei dem das Oxidationsmittel bei der Anwendung mit der Zusammensetzung, wie sie in einem der Ansprüche 1 bis 10 definiert ist, gemischt wird.

15. Verfahren nach Anspruch 12 oder 13, bei dem das Oxidationsmittel in Form einer oxidierenden Zusammensetzung gleichzeitig mit der Zusammensetzung, wie sie in einem der Ansprüche 1 bis 10 definiert ist, oder danach auf die Fasern aufgebracht wird.

16. Vorrichtung mit mehreren Compartments oder "Kit" zum Färben mit mehreren Compartments, wobei ein erstes Compartment eine Zusammensetzung, wie sie in einem der Ansprüche 1 bis 10 definiert ist, und ein zweites Compartment eine oxidierende Zusammensetzung enthält.

17. Verbindung der Formel (I) wie in einem der Ansprüche 1 bis 7 definiert.

18. Verwendung der in den Ansprüchen 1 bis 11 definierten Zusammensetzung zum Färben von Keratinfasern.
